# EUROPEAN PATENT SPECIFICATION

(11) **EP 3 643 281 B1**
(45) Date of publication and mention of the grant of the patent: **05.01.2022**
(21) Application number: 18819647.1
(22) Date of filing: 22.05.2018
(51) Int. Cl.: A61F 13/533, A61F 5/455

(54) **ABSORBENT ARTICLE**
ABSORBIERENDER ARTIKEL
ARTICLE ABSORBANT

(30) Priority: 19.06.2017 JP 2017119743
(43) Date of publication of application: 29.04.2020
(73) Proprietor: Daio Paper Corporation, Ehime 799-0492 (JP)
(72) Inventor: TOKUNAGA, Sachiko, Sakura-shi Tochigi 329-1411 (JP)
(74) Representative: Epping - Hermann - Fischer
(86) International application number: PCT/JP2018/019689
(87) International publication number: WO 2018/235498

(56) References cited:
- EP-A1- 2 612 635
- EP-A1- 3 409 249
- JP-A- 2007 195 665
- JP-A- 2011 156 070
- JP-A- 2012 050 699
- JP-A- 2012 120 792
- JP-A- 2012 120 792
- JP-A- 2012 125 625
- JP-A- 2017 047 004

## Description

### Technical Field

The present invention relates to an absorbent article.

### Background Art

Conventionally, in an absorbent article such as a sanitary napkin, a panty liner and an incontinence pad, it is known to form linear compressed grooves in an absorbent article main body for the purpose of controlling a transfer of absorbed body fluid or deforming an absorbent article main body following a shape of a human body.

For example, Patent Literature 1 discloses an absorbent article including: a front compressed groove extending in a width direction in a front region; and a pair of longitudinal compressed grooves extending longitudinally in both side portions which longitudinally extend in an area facing the excretory portion. The front compressed groove is connected with each of the pair of longitudinal compressed grooves, forming a constricted portion inwardly constricted in the width direction, and the front compressed groove has a rearwardly depressed portion in a middle part in the width direction.

Patent Literature 2 discloses an absorbent article including: blood discharge opening corresponding embosses formed on each side of a longitudinal centerline of the absorbent article, the blood discharge opening corresponding embosses each being curved in an arc shape having a center of curvature on the longitudinal centerline side; and a front emboss formed in a front side portion located forward of and spaced apart from the blood discharge opening corresponding embosses, the front emboss extending approximately along a width direction of the absorbent article; and a rear emboss formed in a rear side portion located rearward of and spaced apart from the blood discharge opening corresponding embosses, the rear emboss having a substantial inverted V-shape which rearwardly gradually expands from the longitudinal centerline to both sides.

EP3409249A1 relates to an absorbent article such as a sanitary napkin, a panty liner, an incontinence pad, and a disposable diaper for absorbing body fluids such as menstrual blood, vaginal discharge, and urine and specifically, relates to an absorbent article having a protruding part that fits a intergluteal cleft formed by deforming compressed grooves formed in a surface and an outline of a rear end of the absorbent article while associating the compressed grooves with the outline of the rear end of the absorbent article.

EP2612635A1 relates to an elongated shaped absorbent article including: a liquid permeable top sheet that is disposed on a wearer's skin side and has a skin contacting surface; a liquid impermeable back sheet that is disposed on an underwear side; and a liquid absorptive absorbent core that is disposed between the top sheet and the back sheet, wherein:
a compressed groove is formed on the skin contacting surface;
the skin contacting surface has an excretory contact region that is positioned in a substantially central portion of the absorbent article in a longitudinal direction and in contact with the vicinity of the excretory part of the wearer, a front region that is positioned more on a front side than the excretory contact region, and a rear region that is positioned more on a rear side than the excretory contact region;
the compressed groove has a pair of first central grooves that are arranged along the longitudinal direction of the absorbent article in the excretory contact region and a first circular groove that is provided in at least any one of the front region and the rear region; and
the first circular groove is arranged at a position overlapping a vertical center line that extends in the longitudinal direction of the absorbent article.

JP2012120792A relates to an absorbent article that absorbs women's menstrual blood, vaginal discharge, and the like, and more particularly, to an absorbent article in which an absorbent element is raised three-dimensionally so as to be closely attached to a wearer's excretory part.

### Citation List

### Patent Literature

Patent Literature 1: Patent Publication No. 6042312
Patent Literature 2: Patent Publication No. 5349283
Patent Literature 3: EP3409249A1
Patent Literature 4: EP2612635A1
Patent Literature 5: JP2012120792A

### Summary of Invention

### Technical Problem

Patent Literature 1 discloses that the depressed portion of the front compressed groove becomes a base point at which the absorbent article can be easily bent, thereby less likely generating longitudinal wrinkles in the front region. However, in the structure disclosed in Patent Literature 1, the portion protruded by bending at the depressed portion as a base point may abut against the skin, and thus comfortable fit of the absorbent article may possibly be impaired.

In addition, in Patent Literature 2, it is described that the fittability of the absorbent article following the shape and the movement of the human body is improved with the disclosed structure. However, in the absorbent article of Patent Literature 2, the fittability of the end region along the roundness of the human body may not be sufficient.

In view of the above, it is an object of the present invention to provide an absorbent article in which the end region can be well fitted along the curvature of the human body, and which can provide a remarkably comfortable fit.

### Solution to Problem

The present invention provides an absorbent article as defined in claim 1.

### Advantageous Effects of Invention

According to the present invention, an absorbent article is provided in which the end region can be well fitted along the curvature of the human body, and which can provide a remarkably comfortable fit.

Embodiments of the present invention will be described as follows.

### (Embodiment 1)

An embodiment includes a main body including a liquid-permeable topsheet, a liquid-impermeable backsheet, and an absorbent body disposed between the topsheet and the backsheet, wherein the main body has an elongated shape with a predetermined length in a front-rear direction and a predetermined width in a width direction orthogonal to the front-rear direction, the main body having a middle region including a body fluid discharge opening corresponding region that corresponds to a body fluid discharge opening of the wearer when attached, a front end region forward of the middle region, and a rear end region rearward of the middle region, the main body having a compressed groove recessed from a topsheet side to a backsheet side, wherein the compressed groove includes: a pair of front-rear direction compressed grooves extending in the front-rear direction; and an end region compressed groove formed in at least one of the front end region and the rear end region, and having a shape including a substantial V-shape projecting toward the body fluid discharge opening corresponding region, and wherein the front-rear direction compressed grooves each have a portion extended beyond a tip of the substantial V-shape of the end region compressed groove in a direction away from the body fluid discharge opening corresponding region, and the portions extended beyond the tip of the substantial V-shape of the end region compressed groove are formed closer to the front-rear direction centerline toward their end edges.

The embodiment 1 provides that the end region compressed groove is formed in at least one end region, and has a shape including a substantial V-shape. Therefore, the tip portion of the end region compressed groove becomes a base point, at which the end region of the absorbent article main body is easily curved in the front-rear direction, and also in the width direction around the two grooves extending from the tip of the substantial V-shape. In addition, the front-rear direction compressed grooves extend beyond the location of the tip of the substantial V-shape of the end region compressed groove, and thus become base points at which the lateral portions of the end region are deformed. Accordingly, the end region of the main body can be deformed along the roundness of the human body, allowing the main body to be well fitted to the human body to improve wearing comfort.

### (Embodiment 2)

In the embodiment 2, at least one end portion of the front-rear direction compressed grooves is curved inwardly in the width direction.

The embodiment 2 provides that the end of the front-rear direction compressed groove is curved inwardly in the width direction toward the end region compressed groove. This allows a region laterally forward of and/or a region laterally rearward of the front-rear direction compressed grooves to be easily bent toward the human body, and thus the absorbent article main body to be well fitted along the body curvature.

### (Embodiment 3)

In the embodiment 3, a distance in the front-rear direction between the tip of the substantial V-shape of the end region compressed groove, and an edge of the portion of the front-rear direction compressed grooves extended beyond the tip of the substantial V-shape is 2.0 mm to 8.0 mm.

The embodiment 3 provides that the distance in the front-rear direction between the location of the tip of the end region compressed groove and the location of the edge of the extended portion of the front-rear direction compressed groove beyond the location of the tip of the substantial V-shape of the end region compressed groove is defined in a predetermined range. This enables to prevent the end region from having an excessive rigidity induced by the front-rear direction compressed groove and thus causing discomfort when attached, while maintaining the function of the front-rear direction compressed groove as a base point for curving.

### (Embodiment 4)

In the embodiment 4, the front-rear direction compressed groove includes high-compressed portions, and a low-compressed portion having a shallower depression than the high-compressed portions, and the portion of the front-rear direction compressed groove extended beyond the tip of the substantial V-shape of the end region compressed groove does not include any of the high-compressed portions.

The embodiment 4 provides that the portion of the front-rear direction compressed groove extended beyond the location of the tip of the substantial V-shape of the end region compressed groove does not have the high-compressed portion. This allows the portion of the front-rear direction compressed groove extended beyond the tip of the end region compressed groove to be relatively less rigid. Therefore, an excessive rigidity in the end region can be prevented and thus discomfort when attached to the skin can be eliminated, while maintaining the function of the front-rear direction compressed groove as the base point for curving

### (Embodiment 5)

In the embodiment 5, the substantial V-shape has an angle of 60 to 150 degrees.

The embodiment according to Appendix 5 provides that the angle of the letter V of the V-shape in the end region compressed groove may be a predetermined angle. This allows a natural deformation of the main body along the roundness of the human body.

### (Embodiment 6)

In the embodiment 6, the front-rear direction compressed grooves and the end region compressed groove are spaced apart in the width direction.

The embodiment 6 provides that the front-rear direction compressed grooves and the end region compressed groove are formed spaced apart. This allows for preventing the compressed grooves being densely arranged and thus excessively rigid.

### (Embodiment 7)

In the embodiment 7, the end region compressed groove is formed in the front end region, and the front-rear direction compressed grooves are connected to each other in the rear end region.

The embodiment 7 provides that the end region compressed groove is formed in the front end region, and the paired front-rear direction compressed grooves are connected to each other in the rear end region. Therefore, the pair of the front-rear direction compressed grooves is to be a closed form in the rear end region, so that the rearward leakage can be well prevented while maintaining the effect of the first embodiment in the front end region of the absorbent article main body.

### Brief Description of Drawings

FIG. 1 is a partially cutaway view of an absorbent article according to one embodiment of the present invention.
FIG. 2 is a cross-sectional view taken along the line I-I of an absorbent article according to one embodiment of the present invention.
Fig. 3 is an enlarged view of the front end region of Fig. 1.
Fig. 4 is an enlarged view of the front end region of Fig. 1.
FIG. 5 is a view illustrating variations of the end region compressed groove in one embodiment of the present invention.
FIG. 6 is a view of an absorbent article according to another embodiment of the invention.

### Description of Embodiments

Embodiments of the present invention will now be described referring to the drawings.

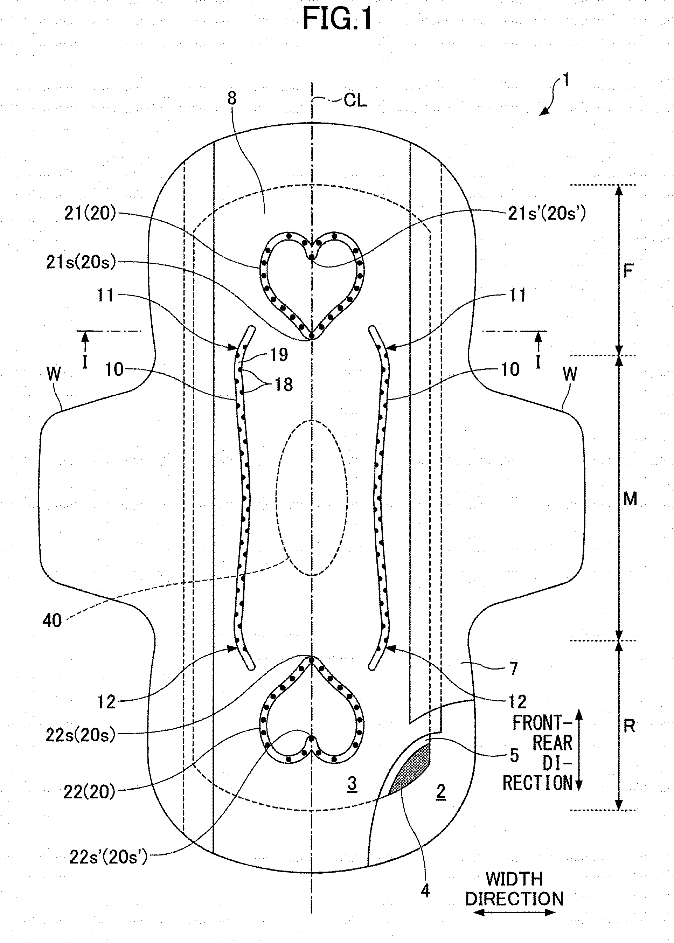

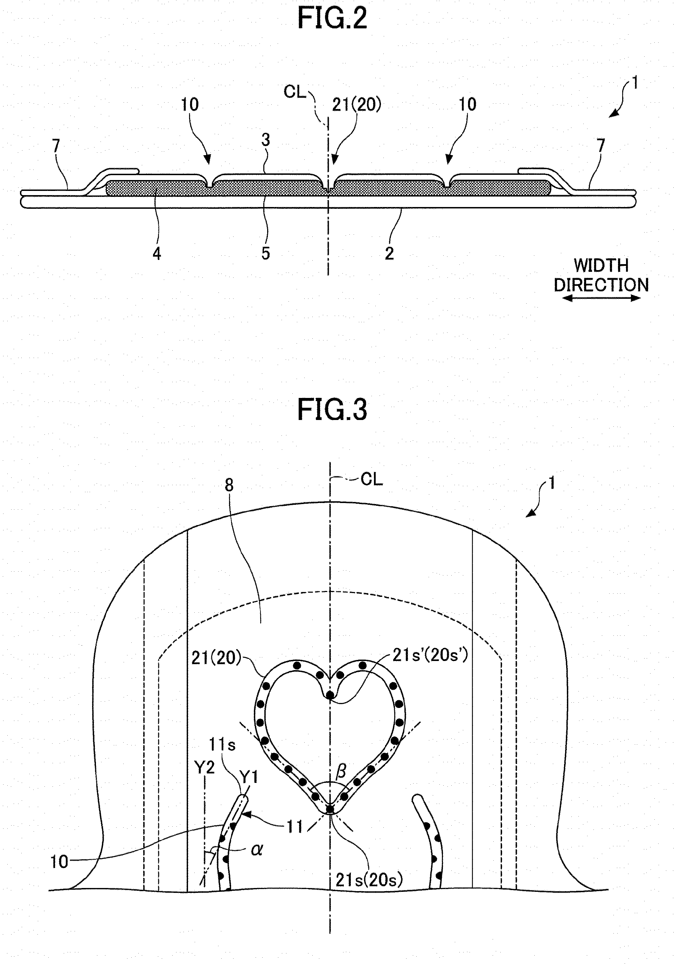

Fig. 1 shows a partially cutaway view of an absorbent article 1 according to an embodiment of the present invention. Fig. 2 is a cross-sectional view taken along the line I-I of Fig. 1. As shown in FIGs. 1 and 2, the absorbent article 1 includes a main body (absorbent article main body) 8 having a liquid-impermeable backsheet 2, a liquid-permeable topsheet 3, and an absorbent body 4 disposed between the sheets 2 and 3. In order to maintain the shape of the absorbent body 4, the absorbent body 4 may be wrapped with an enveloping sheet 5 made of crepe paper, nonwoven fabric, or the like.

The main body 8, as shown in FIG. 1, has a substantially elongated shape having a predetermined length in the front-rear direction and having a width that is substantially constant in a direction orthogonal to the front-rear direction. The absorbent article 1 has a substantially line-symmetric shape with respect to a centerline CL extending in the front-rear direction. The absorbent article main body 8 includes a middle region M including a region corresponding to a body fluid discharge opening, such as a vaginal opening of the wearer (a body fluid discharge opening corresponding region) 40, a front end region F forward of the middle region M, and a rear end region R rearward of the middle region M (the front end region F and the rear end region R are also collectively referred to as the end region) . The middle region M may have wings W, W respectively extending from both lateral sides for securing the absorbent article 1 to the underwear when attached. The front end region F may be a region forward of the front end of the wing W, and the rear end region R may be a region rearward of the rear end of the wing W.

At the front and rear ends of the absorbent body 4, the outer edges of the backsheet 2 and the topsheet 3 may be bonded by an adhesive such as a hot-melt adhesive, or by adhesive means such as a heat seal or an ultrasonic seal. Upon each lateral side of the topsheet 3, a side nonwoven fabric 7 is provided along the front-rear direction (the longitudinal direction). The side nonwoven fabric 7 is partially protruded laterally from the main body 8, and laminated to the backsheet 2, which is also protruded laterally from the main body 8. The protruded nonwoven fabric 7 and the backsheet 2 may be bonded by an adhesive such as a hot-melt, or by adhering means such as a heat seal or an ultrasonic seal, thereby forming wings W, W on the both lateral sides of the main body 8.

The backsheet 2 may be made of a sheet material having at least water-shielding properties. For example, a resin sheet of an olefin such as polyethylene or polypropylene may be used. A laminated nonwoven fabric, which is formed by laminating a nonwoven fabric on a polyethylene sheet or the like, or a laminated sheet of nonwoven fabric layers which has a waterproof film interposed therein to secure substantial liquid-impermeability may also be used. Further, a moisture-permeable sheet is preferably used in order to prevent stuffiness. An example of such water-shielding/moisture-permeable sheet material includes a microporous sheet formed by melt-kneading an olefin resin such as polyethylene or polypropylene with an inorganic filler to form a sheet, and then stretching the sheet in a uniaxial direction or biaxial directions.

The topsheet 3 is a liquid-permeable sheet that allows quick passage of body fluid such as menstrual blood, vaginal discharge, or urine. As the topsheet 3, a porous or nonporous nonwoven fabric or a porous plastic sheet is preferably used. Examples of fiber materials for the nonwoven fabric may include synthetic fibers of an olefin such as polyethylene or polypropylene, a polyester, a polyamide, or the like; regenerated fibers of rayon, cupra, or the like; blended fibers of these; and natural fibers of cotton, or the like. These fibers may be used alone or in any combination. Further, methods of processing the nonwoven fabric may include spunlacing, spunbonding, thermal bonding, melt blowing, and needle punching. Among these processing methods, the spunlacing is preferable in being capable of manufacturing a nonwoven fabric with good flexibility, the spunbonding is preferable in being capable of manufacturing a nonwoven fabric with good drapability, and the thermal bonding is preferable in being capable of manufacturing a bulky, soft nonwoven fabric. Further, composite fibers such as sheath-core fibers having a high melting point fiber as a core and a low melting point fiber as a sheath, side-by-side fibers, and split fibers may be used.

The material for the absorbent body 4 interposed between the backsheet 2 and the topsheet 3 is not particularly limited as long as being capable of absorbing and retaining body fluids, but for example, the absorbent body 4 preferably includes cotton-like pulp and a water absorptive polymer. Examples of the water absorptive polymer may include superabsorbent polymer (SAP), superabsorbent fiber (SAF), and combinations thereof. Examples of pulp include cellulose fibers such as chemical pulp obtained from wood, dissolving pulp, and the like, and also artificial cellulose fibers of rayon, acetate, or the like. Hardwood materials, softwood materials, and the like may be used as the raw material for the chemical pulp, but the softwood materials are preferably used due to their long fiber length, etc.

A synthetic fiber may be mixed in the absorbent body 4. Examples of materials used for the synthetic fiber may include polyolefins such as polyethylene or polypropylene, polyesters such as polyethylene terephthalate or polybutylene terephthalate, polyamides such as nylon, and copolymers thereof. Two or more of these materials may also be used in mixture. Further, multi-component fibers such as sheath-core fibers having a core fiber with a high melting point and a sheath fiber with a low melting point, side-by-side fibers, split fibers, and the like may also be used. Hydrophobic fiber subjected to a surface treatment with a hydrophilizing agent to exhibit affinity to body fluid may be used.

The thickness of the absorbent body 4 is within the range from 0.5 mm to 25 mm, preferably within the range from 1.5 mm to 6.5 mm. The absorbent body 4 may not necessarily have a uniform thickness across its entire surface. For example, the absorbent body 4 may have a bulging in the body fluid discharge opening corresponding region 40 and the vicinity thereof, or in the portion corresponding to the intergluteal cleft (intergluteal cleft corresponding portion) and the vicinity thereof. Further, the absorbent body 4 is preferably manufactured by a stacking process or an air laid process.

Examples of materials used for the side nonwoven fabric 7 include a water-repellent treated nonwoven fabric, and a hydrophilically treated nonwoven fabric. For example, for enhancing the anti-permeation effect against menstrual blood, vaginal discharge, or the like, or improving the feel of texture, a water-repellent treated nonwoven fabric coated with a silicone, paraffin, or alkyl chromic chloride water repellent, or the like may be used. For enhancing absorbability of menstrual blood or the like at the wing W, a hydrophilically treated nonwoven fabric may be used as the material of the nonwoven fabric. A preferable type of the nonwoven fabric is an air-through nonwoven fabric that is less likely to develop folds, wrinkle-resistant, and soft.

On the outer edge of the wings W, W, for the purpose of bonding the side nonwoven fabric 7 and the backsheet 2, and increasing rigidity, dotted compressed portions (embosses) or a compressed portion with a predetermined shape may be provided in a predetermined area.

The total length of the absorbent article 1 may be 130 to 450 mm, preferably 140 to 360 mm, and more preferably 170 to 270 mm.

The absorbent article 1 is provided with compressed grooves (also called pressed grooves or embosses). The compressed groove is formed as a linear groove depressed from the topsheet 3 toward the backsheet 2. The compressed groove may function to prevent the absorbent body 4 to be twisted, to control a transfer of body fluid on the surface of or inside of the absorbent body, and to facilitate a deformation following the shape of the human body. The compressed groove can be formed by passing a stack of the topsheet 3 and the absorbent body 4 between a pair of pressing rolls. For example, the stack may be passed between rolls so that a roll with protrusions is arranged on the topsheet 3 side and a flat roll with no protrusion arranged on the absorbent body 4 side.

The compressed groove includes a pair of front-rear grooves 10, 10 extending in the front-rear direction, and line-symmetrically with respect to the front-rear direction centerline CL of the main body, as shown in FIG. 1. The pair of front-rear direction compressed grooves 10, 10 may extend along the lateral sides of the body fluid discharge opening corresponding region 40, or through a location laterally spaced apart from the body fluid discharge opening corresponding region 40 by a predetermined distance, so that the grooves 10, 10 do not extend through the body fluid discharge opening corresponding region 40. The front-rear direction compressed grooves 10, 10 may be provided mainly in the middle region M, and may extend beyond a front end and a rear end of the middle region M, as shown in FIG. 1. The front-rear direction compressed grooves 10, 10 may extend linearly as a whole, or may be curved in mid-course as illustrated example. The pair of longitudinal grooves 10, 10 may not necessarily be linear-symmetrically provided. However, the paired front-rear grooves 10, 10 are preferably provided on each side of the front-rear direction centerline CL.

The pair of longitudinal compressed grooves 10, 10 facilitates bending of the main body 8 of the absorbent article 1, as they become base points (flexible shafts) for the bending. Therefore, the absorbent article 1 is attached and the main body 8 is subjected to a force laterally from both legs, and the area between the front-rear direction compressed grooves 10, 10 is easily raised toward the human body side, thereby increasing the fittability of the middle region M to the human body.

The compressed groove of the absorbent article 1 includes an end region compressed groove 20 formed in at least one of the front end region F and the rear end region R. That is, the end region compressed groove 20 includes the front end region compressed groove 21 formed in the front end region F and/or the rear end region compressed groove 22 formed in the rear end region R. In the example of FIG. 1, the front end region compressed groove 21 and the rear end region compressed groove 22 are both formed.

The end region compressed groove 20 has a shape that includes a substantial V-shape projecting toward the body fluid discharge opening corresponding region 40. In the example of FIG. 1, the front end region compressed groove 21 has a heart-shape including a pointed tip 21s of a V-letter projecting toward the body fluid discharge opening corresponding region 40, and further includes a pointed tip 21s' of a V-letter formed forward of the tip 21s also projecting toward the body fluid discharge opening corresponding region 40. The rear end region compressed groove 22 has an inverted heart-shape including a pointed tip 22s of a V-letter projecting toward the body fluid discharge opening corresponding region 40, and a pointed tip 22s' of a V-letter formed rearward of the tip 22s also projecting toward the body fluid discharge opening corresponding region 40. Thus, in the illustrated example, the forward region compressed groove 21 and the rear region compressed groove 22 each have a shape including a plurality of V-shaped portions.

In the example of FIG. 1, the absorbent article 1 is bilaterally symmetric and also fore-aft symmetric (vertically symmetric in the drawing) . That is, when one of the front end region compressed groove 21 and the rear end region compressed groove 22 is inverted in the front-rear direction and superimposed on the other, the size and the shape of the superimposed grooves are the same. However, the absorbent article 1 may not necessarily be fore-aft symmetric. The front end region compressed groove 21 and the rear end region compressed groove 22 may be different in size, and may also not necessarily be formed in a fore-aft symmetric shape. Further, the configuration (including the shape) of the absorbent article 1 or the main body 8 may not necessarily be bilaterally symmetric.

Since the end region compressed groove 20 has a shape including a substantial V-shape, it is easy to bend the end region at the V-shaped portion, which serves as a base point or a base edge for bending, both in the front rear direction and in the width direction, when attached. This allows the end region of the main body 8, which is planar before attached, to be deformed in a cup-like shape to form a curved surface. Thus, the end region of absorbent article 1 can be naturally fitted along the roundness of the human body at the front and/or rear of the body. Therefore, the absorbent article 1 can follow the body even during walking or exercise, and discomfort when attached can be eliminated. Further, it is possible to prevent an excessive force from being applied to a portion of the main body 8 that would not be fitted to the human body, which would cause twists or the like.

The tip 20s of the V-shaped portion including the above mentioned V-letter of the end compressed groove 20 is preferably formed on the front-rear direction centerline CL, so that the end region(s) can be bilaterally well balanced and curved.

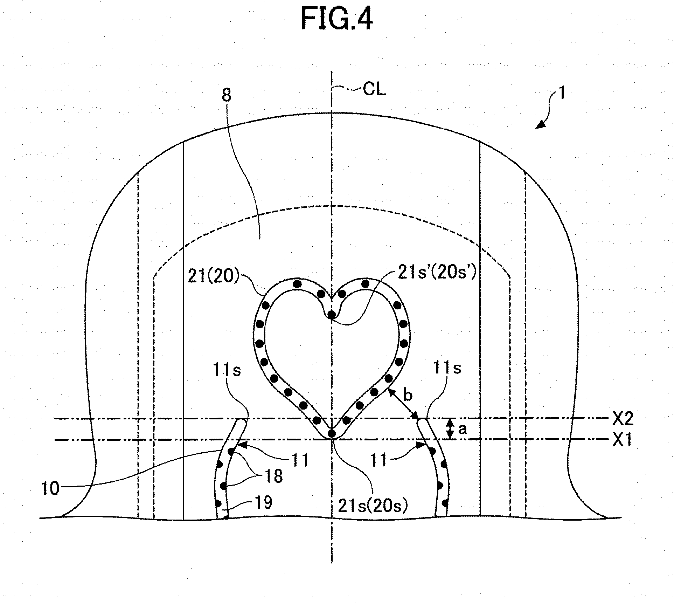

Further, in the present example, as shown in FIGs. 1 and 4, the front-rear direction compressed grooves 10, 10 extend beyond the location of the tip 20s of the substantial V-shape of the end region compressed groove 20 in a direction away from the body fluid discharge opening corresponding region 40. In other words, as shown in Fig. 4, when the virtual line X1 extending in the width direction through the tip 20s of the end region compressed groove 20 is drawn, the front-rear direction compressed grooves 10, 10 extend beyond the virtual line X1 in a direction away from the middle region M.

In this case, the front-rear direction compressed grooves 10, 10 may extend in the front end region F beyond the location of the tip 21s of the substantial V-shape of the front end region compressed groove 21 away from the body fluid discharge opening corresponding region 40, or may extend in the rear end region R beyond the location of the tip 22s of the substantial V-shape of the rear end region compressed groove 22 away from the body fluid discharge opening corresponding region 40. It is preferable, as shown in the illustrated example, that front end portions 11, 11 of the front-rear direction compressed grooves 10, 10 extend beyond the location of the tip 21s of the V-shape of the front end region compressed groove 21 formed in the front end region F in a direction away from the body fluid discharge opening corresponding region 40 (toward the front of main body 8), and the rear end portions 12, 12 of the front-rear direction compressed grooves 10, 10 extend beyond the location of the tip 22s of the V-shape of the front end region compressed groove 22 formed in the rear end region R in a direction away from the fluid drain corresponding region 40 (toward the rear of main body 8).

With the above-described arrangement, the front-rear direction compressed grooves 10, 10 can also become base points or base edges of deformation of the main body 8 in the end region. Specifically, on the virtual line X1 shown in FIG. 4, three locations, i.e., the locations of the tip 21s of the V-shape of the front end region compressed groove 21, and the front end portions 11, 11 of the pair of front-rear direction compressed grooves 10, 10 may become the base points for folding. Further, on a virtual line X2, four locations, i.e., locations of each branched compressed groove which are slightly forward of the tip 21s of the V-shape of the front end region compressed groove 21, and the pair of front-rear direction compressed grooves 10, 10 may become the base points for folding. Such multiple points for folding in the end region allow the planar main body 8 to be more naturally curved. Therefore, the front end region F is well fitted to the curve of pubic bone of the wearer, and the rear end region R is well fitted to the curve of buttocks of the wearer, thereby enhancing comfortable fit and preventing leakage of body fluid from lateral sides of the end region compressed groove 20.

Further, as shown in FIG. 1, edges of the extended portions of the front-rear direction compressed grooves 10, 10 extended beyond the location of the tip 20s of the substantial V-shape of the end region compressed groove 20 are curved or inclined inwardly in the width direction. In other words, the front-rear direction compressed grooves 10, 10 are formed closer to the front-rear direction centerline toward their end edges.

The end portions of the front-rear direction compressed grooves 10, 10 may extend substantially parallel to the front-rear direction centerline CL, or may be curved or bent, inwardly or outwardly in the width direction. However, the end portions of the front-rear direction compressed grooves 10, 10 are preferably bent inwardly in the width direction.

In this case, either of the front end portions 11, 11 or the rear end portions 12, 12 of the front-rear direction compressed grooves 10, 10 may be curved inwardly in the width direction. However, the front end portions 11, 11 of the front-rear direction compressed grooves 10, 10 are preferably curved inwardly in the width direction. It is also preferred, as shown in the illustrated example, that the front end portions 11, 11 of the front-rear direction compressed grooves 10, 10 are curved inwardly in the width direction in the front end region F, and the rear end portions 12, 12 of the front-rear direction compressed grooves 10, 10 are curved inwardly in the width direction in the rear end region R. The front end portions 11, 11 of the front-rear direction compressed grooves 10, 10 may be defined as portions having some length including front edges of the front-rear compressed grooves 10, 10, and the rear end portions 12, 12 may defined as portions having some length including rear edges of the front-rear compressed grooves 10, 10.

As described above, the end portions of the front-rear direction compressed grooves 10, 10 are bent inwardly in the width direction, which allows the lateral region forward of the front end portions of the front-rear direction compressed grooves 10, 10 and/or the lateral region rearward of the rear end portions of the front-rear direction compressed grooves 10, 10 to be bend more easily toward the human body when the end regions are placed onto the body when attached. Therefore, the main body 8 can be better fitted along the roundness of the body. Further, the leakage of the body fluid from the lateral sides of the end region compressed groove 20 can be better prevented.

The degree of curvature of the end of the front-rear direction compressed groove 10 is not particularly limited, but the angle α between the direction of extension of the curved end portion and the front-rear direction centerline CL is preferably 10 to 80 degrees, more preferably 10 to 50 degrees, and more preferably 15 to 40 degrees. The above range of the angle α further improves the aforementioned effect that the end region is curved conforming along the body roundness to improve the fittability.

The angle α may be, for example, an angle between a tangent line of the centerline of the front-rear direction compressed groove 10 (groove centerline) which extends orthogonal to the groove width, and the front-rear direction centerline CL. The angle α may also be a maximum value of the angle between the tangent line of the groove centerline of the front-rear direction compressed groove 10 and the front-rear direction centerline CL.

FIG. 3 is an enlarged view mainly showing the front part of the absorbent article 1 of FIG. 1. In the example of FIG. 3, the angle α is shown as an angle between the tangent line Y1 of the groove centerline at the end portion 11 of the front-rear direction compressed groove 10 and a line Y2 parallel to the front-rear direction centerline CL.

An angle β of the V-shape of the end region compressed groove 20 is also not particularly limited, but may be preferably 60 to 150 degrees, more preferably 90 to 130 degrees, and even more preferably 100 to 120 degrees. The angle β in the above range allows the deformation of the end region to be adapted to the roundness of the human body.

When the V-shaped portion included in the end region compressed groove 20 is formed of two straight grooves connected to each other, the angle β may be an angle between the groove centerlines of the two grooves. Further, when the V-shaped portion included in the end region compressed groove 20 is formed of two curved grooves connected to each other, the angle β may be an angle between the tangent lines of the groove centerlines of the two grooves.

In the illustrated example, the tip 20s of the V-shape of the end region compressed groove 20 is formed by connecting two compressed grooves. However, the two compressed grooves may not necessarily be connected, as long as the aforementioned effect can be provided that the tip 20s becomes a base point for the deformation of the main body conforming to the front and/or rear of the human body to improve fittability. However, even in such a case, it is preferable that the location where extended lines of the groove centerlines of the two compressed grooves are connected may be on the front-rear direction centerline CL.

In the same manner to FIG. 3, FIG. 4 is an enlarged view mainly showing the front part of the absorbent article 1 of FIG. 1. In Fig. 4, a virtual line X1 extends in the width direction through the tip 21s of the V-shape of the front end region compressed groove 21, and a virtual line X2 extends in the width direction through the front edges (terminals) 11s, 11s of the front-rear direction compressed grooves 10, 10. A distance between the virtual line X1 and the virtual line X2 is represented by the letter a. That is, the distance a is a distance in the front-rear direction between the location of the tip 21s of the front region compressed groove 21 and the location of the edges 11s, 11s of the front end portions 11, 11 of the front-rear region compressed grooves 10, 10.

The distance a is preferably 2.0 to 8.0 mm, and more preferably 3.0 to 6.0 mm. The distance a of 2.0 mm or more enables to improve the function of the front-rear direction compressed grooves 10, 10 as the base points for curving. Further, the distance a of 8.0 mm or less enables to prevent excessive rigidity in the end region induced by the front-rear direction compressed grooves 10, 10, thereby preventing discomfort when attached.

The front-rear direction compressed grooves 10, 10 may include high-compressed portions 18 and a low-compressed portion 19 having a shallower depression than the high-compressed portions 18 (FIGs. 1 and 4). In the drawings, the high-compressed portions 18 are illustrated in black and the low-compressed portion 19 is illustrated in white (colorless). The depths of compression of the high-compressed portions 18 and the low-compressed portion 19 are not particularly limited. However, the high-compressed portions 18 may be formed to have a groove depth of approximately 0.5 to 3 mm deeper than the bottom surface of the low-compressed portion 19. Such a configuration including high-compressed portions and a low-compressed portion can be obtained by using pressure rolls having recesses and protrusions corresponding to a desired groove structure. For example, a pressure roll may be used which is configured to have protruded portions corresponding to the shape of the entire compressed groove, and fine protrusions corresponding to the high-pressing portions are provided on the protruded portions. Alternatively, the low-compressed portions may be firstly formed at a relatively low pressure in the region where the compressed grooves are desired to be formed, and then the high-compressed portions may be formed partially in the region of the low-compressed portions. The depth of the compressed portion can be adjusted by adjusting the pressure of the above-described pressure rolls used for forming the compressed grooves.

When the front-rear direction compressed grooves 10, 10 have high-compressed portions 18 and a low-compressed portion 19, the extended portions of the front-rear direction compressed grooves 10, 10 extended beyond the location of the tip 20s of the V-shape of the end region compressed groove 20 preferably do not have any of the high-compressed portions 18. That is, as shown in FIG. 3, it is preferable that the front-rear direction compressed grooves 10, 10 do not have high-compressed portions 18 in the area between the virtual line X1 and the virtual line X2 in the front-rear direction.

The high-compressed portions 18 are more intensively compressed portions. Therefore, when the high-compressed portions 18 are provided, or when the high-compressed portions 18 are densely provided, the rigidity of the portion may be increased and the flexibility of the area surrounding the compressed groove may be reduced. Therefore, by not providing the high-compressed portions 18 in the portions of the front-rear direction compressed grooves 10, 10 extended beyond the location of the tip 20s of the V-shape of the end region compressed grooves 20, it is possible to prevent flexibility loss in the areas near and surrounding the end portions of the front-rear direction compressed grooves 10, 10. Therefore, discomfort caused by contact with the end portions of the front-rear direction compressed grooves 10, 10 and their surrounding areas can be reduced or prevented, while maintaining the function that the end portions of the front-rear direction compressed grooves 10, 10 can be the base points for curving.

In the illustrated example, the extended portions of the end region compressed grooves 10, 10 extended beyond the tip 20s of the V-shape of the end region compressed groove 20 do not have any of the high-compressed portions 18. However, the rigidity of the area between the virtual line X1 and the virtual line X2 of the front-rear direction compressed grooves 10, 10 can be reduced by reducing the size of the high-compressed portions 18 or by reducing the pitch between the high-compressed portions 18. Such configurations may also contribute to reducing or preventing the discomfort.

The configuration including the high-compressed portions and the low-compressed portion may also be provided in the compressed grooves other than the front-rear direction compressed grooves 10, 10. In the illustrated example, the end region compressed groove 20 (the front end region compressed groove 21 and the rear end region compressed groove 22) is also provided with high-compressed portions and a low-compressed portion.

The front-rear direction compressed grooves 10, 10 and the end region compressed groove 20 may be connected or spaced apart. However, when the front-rear direction compressed grooves 10, 10 and the end region compressed grooves 20 are spaced apart, excessive rigidity in the lateral side portions of the end region can be avoided. This reduces the discomfort in the end region when attached.

In FIG. 4, the distance between the front-rear direction compressed grooves 10, 10 and the end region compressed groove 20 is represented by letter b. The distance b is the shortest distance between the front-rear direction compressed grooves 10, 10 and the end region compressed groove 20. The distance b is preferably 5 to 15 mm, and more preferably 7 to 10 mm.

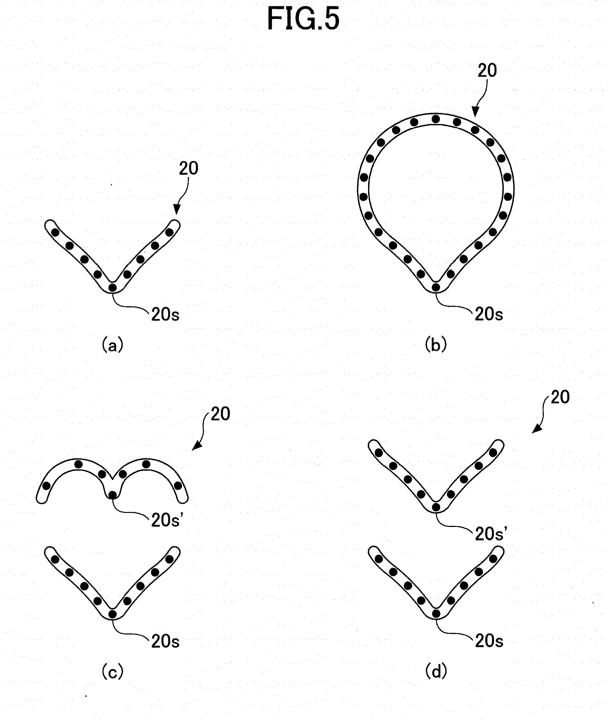

In the illustrated example, the shape including the substantial V-shape of the front end region compressed groove 21 is a heart-shape formed of a single annulus compressed groove, but the shape is not limited to the illustrated one. For example, the shape of the front end region compressed groove may be a V-shape as shown in FIG. 5(a), or a droplet shape as shown in FIG. 5(b). Further, as shown in Fig. 5(c), the shape may be a heart shape as a whole, but may be separated in the front-rear direction. Moreover, as shown in Fig. 5(d), the shape may be an array of a plurality of V-shapes in the front-rear direction.

The shape including a plurality of V-shapes protruding toward the body fluid discharge opening corresponding region 40 as shown in FIGs. 1, 3, 4, and 5(c) and (d) may be preferable, because a plurality of the base points for curving can be formed in the front-rear direction and the main body 8 can be deformed along the body roundness. Figs. 5(c) and (d) have a plurality of the tips 20s, 20s' of the V-shapes. On the other hand, the shape having one tip 20s projecting toward the body fluid discharge opening corresponding region 40 as shown in FIGs. 5(a) and 5(b) may also preferable from a viewpoint of preventing rough feel or the like, because the configuration may avoid a dense arrangement of the compressed portions and may reduce the rigidity.

As described above, the features of the absorbent article 1 have been described mainly with respect to the front end region F of the main body 8, but same or similar features may also be provided with respect to the rear end region R of the absorbent article 1.

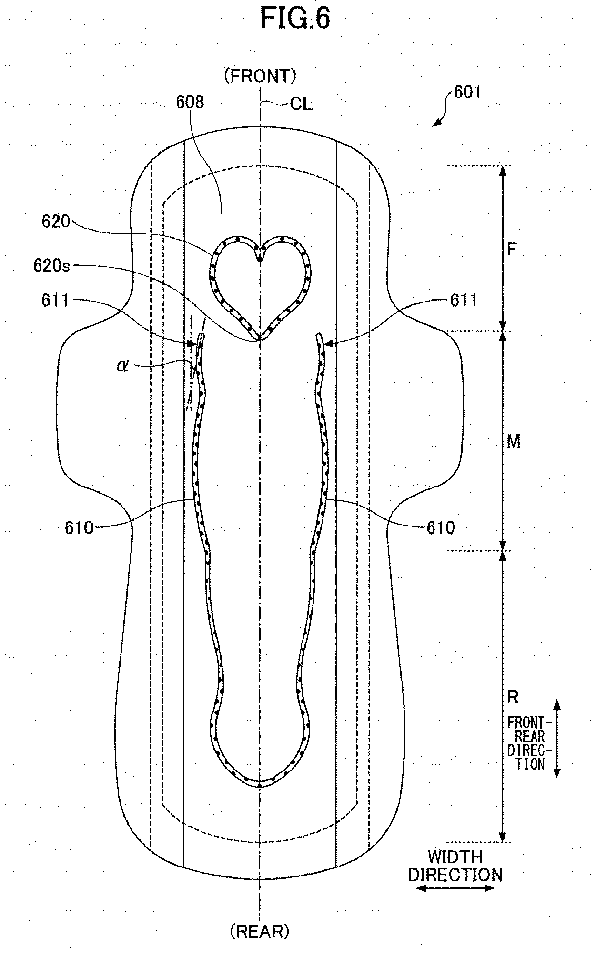

FIG. 6 shows an absorbent article 601 in accordance with another embodiment of the invention. In the same manner as the absorbent article 1, the absorbent article 601 includes a main body 608 including a topsheet 603, an absorbent body, and a backsheet. The absorbent article 601 has a pair of front-rear direction compressed grooves 610, 610 formed line-symmetrically with respect to the front-rear direction centerline CL, and an end region compressed groove 620 having a shape including a substantial V-shape projecting toward the body fluid discharge opening corresponding region 40, the front-rear direction compressed grooves 610, 610 extending beyond the tip 620 of the substantial V-shape of the end region compressed groove 620 in a direction away from the body fluid discharge opening corresponding region 40. However, in the absorbent article 601, the length of the rear end region R and the length of the front-rear direction compressed grooves 610, 610 are respectively longer than those in the absorbent article 1. The end region compressed groove includes a front end region compressed groove 620, and does not include any end region compressed groove in the rear end region F.

Also in the absorbent article 601, the front end portions 611, 611 of the front-rear direction compressed grooves 610, 610 are bent inwardly in the width direction, so that the front end region F can be bent naturally along the pubic curve when attached.

With regard to the degree of bending of the end portion 611 of the front-rear direction compressed groove 610, the angle between the extending direction of the end portion 611 and the front-rear direction centerline CL is preferably 10 to 50 degrees, and more preferably 15 to 30 degrees.

In the absorbent article 601, paired longitudinal compressed grooves 610, 610 are connected to each other at the rear end region R. Such a configuration can prevent rearward leakage. Accordingly, the absorbent article 601 shown in FIG. 6 can be particularly suitably used as a nighttime or long-term sanitary napkin.

This application is based on and claims benefit of priority of Japanese Patent Application No. 2017-119743, filed June 19, 2017.

### Reference Signs List

1, 601 absorbent article
2 backsheet
3, 603 topsheet
4 absorbent article
5 enveloping sheet
7 side nonwoven fabric
8, 608 main body (absorbent article main body)
10, 610 front-rear direction compressed groove
11, 611 front end portion of the front-rear direction compressed groove
11s front edge of the front-rear direction compressed groove
12s rear edge of the front-rear direction compressed groove
12 rear end portion of the front-rear direction compressed groove
18 highly compressed portion
19 lowly compressed portion
20, 620 end region compressed groove
21 front end region compressed groove
22 rear end region compressed groove
20s, 20s', 21s, 21s', 22s, 22s', 620s tip of V-shape
40, 640 body fluid discharge opening corresponding region
CL front-rear direction centerline
F front end region
M middle region
R rear end region
W wing

## Claims

1. An absorbent article (1, 601) comprising a main body (8,608) including a liquid-permeable topsheet (3,603), a liquid-impermeable backsheet (2), and an absorbent body (4) disposed between the topsheet (3, 603) and the backsheet (2),
wherein the main body (8, 608) has an elongated shape with a predetermined length in a front-rear direction and a predetermined width in a width direction orthogonal to the front-rear direction, the main body (8, 608) having a middle region (M) including a body fluid discharge opening corresponding region (40, 640) that corresponds to a body fluid discharge opening of a wearer when attached, a front end region (F) forward of the middle region, and a rear end region (R) rearward of the middle region (M), the main body (8,608) having a compressed groove recessed from a topsheet side to a backsheet side,
wherein the compressed groove includes:
a pair of front-rear direction compressed grooves (10, 610) extending in the front-rear direction; and
an end region compressed groove (20, 620) formed in at least one of the front end region (F) and the rear end region (R), and having a shape including a substantial V-shape projecting toward the body fluid discharge opening corresponding region (40, 640),
wherein the front-rear direction compressed grooves (10, 610) each have a portion extended beyond a tip (20s, 620s) of the substantial V-shape of the end region compressed groove (20, 620) in a direction away from the body fluid discharge opening corresponding region (40, 640), and the portions extended beyond the tip (20s, 620s) of the substantial V-shape of the end region compressed groove (20, 620) are formed closer to the front-rear direction centerline (CL) toward their end edges (11s).

2. The absorbent article (1, 601) according to claim 1, wherein at least one end portion (11, 12, 611) of the front-rear direction compressed grooves (10, 610) is curved inwardly in the width direction.

3. The absorbent article according to claim 1, wherein a distance (a) in the front-rear direction between the tip (20s, 620s) of the substantial V-shape of the end region compressed groove (20, 620), and an edge (11s) of the portion of the front-rear direction compressed grooves (10, 610) extended beyond the tip (20s, 620s) of the substantial V-shape is 2.0 mm to 8.0 mm.

4. The absorbent article (1, 601) according to claim 1, wherein the front-rear direction compressed groove (10, 610) includes high-compressed portions (18), and a low-compressed portion (19) having a shallower depression than the high-compressed portions (18), and
wherein the portion of the front-rear direction compressed grooves (10, 610) extended beyond the tip (20s, 620s) of the substantial V-shape of the end region compressed groove (20, 620) does not include any of the high-compressed portions (18).

5. The absorbent article (1, 601) of claim 1, wherein the substantial V-shape has an angle of 60 to 150 degrees.

6. The absorbent article (1, 601) of claim 1, wherein the front-rear direction compressed grooves (10, 610) and the end region compressed groove (20, 620) are spaced apart in the width direction.

7. The absorbent article (1, 601) of claim 1, wherein the end region compressed groove (20, 620) is formed in the front end region (F), and the pair of front-rear direction compressed grooves (10, 610) are connected to each other in the rear end region (R).

## Patentansprüche

1. Absorbierender Artikel (1, 601) mit einem Hauptkörper (8, 608), der einen flüssigkeitsdurchlässigen oberseitigen Flächenkörper (3, 603), einen flüssigkeitsundurchlässigen rückseitigen Flächenkörper (2) und einen absorbierenden Körper (4) aufweist, der zwischen dem oberseitigen Flächenkörper (3, 603) und dem rückseitigen Flächenkörper (2) angeordnet ist,
wobei der Hauptkörper (8, 608) eine längliche Form mit einer vorbestimmten Länge in einer Vorne-/Hinten-Richtung und einer vorbestimmten Breite in einer Breitenrichtung senkrecht zur Vorne-/Hinten-Richtung hat, wobei der Hauptkörper (8, 608) einen mittleren Bereich (M) hat, der einen einer Körperfluid-Abgabeöffnung entsprechenden Bereich (40, 640), der einer Körperfluid-Abgabeöffnung eines Trägers beim Tragen entspricht, einen vorderen Endbereich (F), der sich vor dem mittleren Bereich befindet, und einen hinteren Endbereich (R) umfasst, der sich hinter dem mittleren Bereich (M) befindet, wobei der Hauptkörper (8, 608) eine zusammengedrückte Rille hat, die von einer oberseitigen Flächenkörperseite zu einer rückseitigen Flächenkörperseite vertieft ist,
wobei die zusammengedrückte Rille Folgendes umfasst:
ein Paar in Vorne-/Hinten-Richtung verlaufende, zusammengedrückte Rillen (10, 610), die sich in der Vorne-/Hinten-Richtung erstrecken; und
eine im Endbereich liegende, zusammengedrückte Rille (20, 620), die im vorderen Endbereich (F) und/oder hinteren Endbereich (R) ausgebildet ist und eine Form hat, die eine allgemeine V-Form umfasst, die zu dem der Körperfluid-Abgabeöffnung entsprechenden Bereich (40, 640) hin vorsteht,
wobei die in Vorne-/Hinten-Richtung verlaufenden, zusammengedrückten Rillen (10, 610) jeweils einen Abschnitt haben, der über eine Spitze (20s, 620s) der allgemeinen V-Form der im Endbereich liegenden, zusammengedrückte Rille (20, 620) hinaus in einer Richtung verlängert ist, die von dem der Körperfluid-Abgabeöffnung entsprechenden Bereich (40, 640) weg führt, und die über die Spitze (20s, 620s) der allgemeinen V-Form der im Endbereich liegenden, zusammengedrückten Rille (20, 620) hinaus verlängerten Abschnitte zu ihren Endrändern (11s) hin so ausgebildet sind, dass sie sich näher an der in Vorne-/Hinten-Richtung verlaufenden Mitteline (CL) befinden.

2. Absorbierender Artikel (1, 601) nach Anspruch 1, wobei zumindest ein Endabschnitt (11, 12, 611) der in Vorne-/Hinten-Richtung verlaufenden, zusammengedrückten Rillen (10, 610) in der Breitenrichtung nach innen gekrümmt ist.

3. Absorbierender Artikel nach Anspruch 1, wobei ein Abstand (a) in der Vorne-/Hinten-Richtung zwischen der Spitze (20s, 620s) der allgemeinen V-Form der im Endbereich liegenden, zusammengedrückten Rille (20, 620) und einem Rand (11s) des Abschnitts der in Vorne-/Hinten-Richtung verlaufenden, zusammengedrückten Rillen (10, 610), die über die Spitze (20s, 620s) der allgemeinen V-Form hinaus verlängert sind, 2,0 mm bis 8,0 mm beträgt.

4. Absorbierender Artikel (1, 601) nach Anspruch 1, wobei die in Vorne-/Hinten-Richtung verlaufende, zusammengedrückte Rille (10, 610) stark zusammengedrückte Abschnitte (18) und einen geringfügig zusammengedrückten Abschnitt (19) umfasst, der eine flachere Vertiefung als die stark zusammengedrückten Abschnitte (18) hat, und
wobei der Abschnitt der in Vorne-/Hinten-Richtung verlaufenden, zusammengedrückten Rillen (10, 610), die bis über die Spitze (20s, 620s) der allgemeinen V-Form der im Endbereich liegenden, zusammengedrückten Rille (20, 620) hinaus verlängert ist, keinen der stark zusammengedrückten Abschnitte (18) umfasst.

5. Absorbierender Artikel (1, 601) nach Anspruch 1, wobei die allgemeine V-Form einen Winkel von 60 bis 150 Grad hat.

6. Absorbierender Artikel (1, 601) nach Anspruch 1, wobei die in Vorne-/Hinten-Richtung verlaufenden, zusammengedrückten Rillen (10, 610) und die im Endbereich liegende, zusammengedrückte Rille (20, 620) in der Breitenrichtung voneinander beabstandet sind.

7. Absorbierender Artikel (1, 601) nach Anspruch 1, wobei die im Endbereich liegende, zusammengedrückte Rille (20, 620) im vorderen Endbereich (F) ausgebildet ist und die beiden in Vorne-/Hinten-Richtung verlaufenden, zusammengedrückten Rillen (10, 610) im hinteren Endbereich (R) miteinander verbunden sind.

## Revendications

1. Article absorbant (1, 601) comprenant un corps principal (8, 608) incluant une feuille supérieure (3, 603) perméable aux liquides, une feuille arrière (2) imperméable aux liquides, et un corps absorbant (4) disposé entre la feuille supérieure (3, 603) et la feuille arrière (2),
sachant que le corps absorbant (8, 608) a une forme allongée avec une longueur prédéterminée dans une direction avant-arrière et une largeur prédéterminée dans une direction de largeur perpendiculaire à la direction avant-arrière, le corps principal (8, 608) présentant une région médiane (M) incluant une région correspondante d'ouverture d'évacuation de fluide corporel (40, 640) qui correspond à une ouverture d'évacuation de fluide corporel d'un porteur lorsqu'il est attaché, une région d'extrémité avant (F) vers l'avant de la région médiane, et une région d'extrémité arrière (R) vers l'arrière de la région médiane (M), le corps principal (8, 608) présentant une rainure comprimée évidée depuis un côté feuille supérieure vers un côté feuille arrière,
sachant que la rainure comprimée inclut :
une paire de rainures comprimées de direction avant-arrière (10, 610) s'étendant dans la direction avant-arrière ; et
une rainure comprimée de région d'extrémité (20, 620) formée dans au moins l'une de la région d'extrémité avant (F) et de la région d'extrémité arrière (R), et ayant une forme incluant une forme sensiblement en V faisant saillie vers la région correspondante d'ouverture d'évacuation de fluide corporel (40, 640),
sachant que les rainures comprimées de direction avant-arrière (10, 610) ont chacune une partie étendue au-delà d'une pointe (20s, 620s) de la forme sensiblement en V de la rainure comprimée de région d'extrémité (20, 620) dans une direction s'éloignant de la région correspondante d'ouverture d'évacuation de fluide corporel (40, 640), et les parties étendues au-delà de la pointe (20s, 620s) de la forme sensiblement en V de la rainure comprimée de région d'extrémité (20, 620) sont formées plus près de la ligne médiane de direction avant-arrière (CL) vers leurs bords d'extrémité (11s).

2. L'article absorbant (1, 601) selon la revendication 1, sachant qu'au moins une partie d'extrémité (11, 12, 611) des rainures comprimées de direction avant-arrière (10, 610) est courbée vers l'intérieur dans la direction de largeur.

3. L'article absorbant selon la revendication 1, sachant qu'une distance (20s, 620s) dans la direction avant-arrière entre la pointe (20s, 620s) de la forme sensiblement en V de la rainure comprimée de région d'extrémité (20, 620) et un bord (11s) de la partie des rainures comprimées de direction avant-arrière (10, 610) étendue au-delà de la pointe (20s, 620s) de la forme sensiblement en V est de 2,0 mm à 8,0 mm.

4. L'article absorbant (1, 601) selon la revendication 1, sachant que la rainure comprimée de direction avant-arrière (10, 610) inclut des parties fortement comprimées (18), et une partie faiblement comprimée (19) ayant un évidement moins profond que les parties fortement comprimées (18), et
sachant que la partie des rainures comprimées de direction avant-arrière (10, 610) étendue au-delà de la pointe (20s, 620s) de la forme sensiblement en V de la rainure comprimée de région d'extrémité (20, 620) n'inclut pas l'une quelconque des parties fortement comprimées (18).

5. L'article absorbant (1, 601) de la revendication 1, sachant que la forme sensiblement en V a un angle de 60 à 150 degrés.

6. L'article absorbant (1, 601) de la revendication 1, sachant que les rainures comprimées de direction avant-arrière (10, 610) et la rainure comprimée de région d'extrémité (20, 620) sont espacées dans la direction de largeur.

7. L'article absorbant (1, 601) de la revendication 1, sachant que la rainure comprimée de région d'extrémité (20, 620) est formée dans la région d'extrémité avant (F), et la paire de rainures comprimées de direction avant-arrière (10, 610) sont connectées l'une à l'autre dans la région d'extrémité arrière (R).
